(19) **Europäisches Patentamt / European Patent Office / Office européen des brevets**

(11) **EP 4 582 088 A1**

(12) **EUROPEAN PATENT APPLICATION**
published in accordance with Art. 153(4) EPC

(43) Date of publication:
09.07.2025 Bulletin 2025/28

(21) Application number: 23859278.6

(22) Date of filing: 28.08.2023

(51) International Patent Classification (IPC):
A61K 31/7068 (2006.01)    A61P 35/00 (2006.01)
A61P 35/04 (2006.01)    A61K 31/136 (2006.01)

(52) Cooperative Patent Classification (CPC):
A61K 31/136; A61K 31/7068; A61P 35/00;
A61P 35/04

(86) International application number:
PCT/CN2023/115149

(87) International publication number:
WO 2024/046246 (07.03.2024 Gazette 2024/10)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 29.08.2022 CN 202211039758

(71) Applicant: CSPC Zhongqi Pharmaceutical
Technology
(Shijiazhuang) Co., Ltd.
Shijiazhuang, Hebei 050035 (CN)

(72) Inventors:
• LI, Chunlei
  Shijiazhuang, Hebei 050035 (CN)
• LIU, Yanping
  Shijiazhuang, Hebei 050035 (CN)
• LI, Mengmeng
  Shijiazhuang, Hebei 050035 (CN)

• HUANG, Wei
  Shijiazhuang, Hebei 050035 (CN)
• DU, Yanling
  Shijiazhuang, Hebei 050035 (CN)
• HE, Lijuan
  Shijiazhuang, Hebei 050035 (CN)
• WANG, Shixia
  Shijiazhuang, Hebei 050035 (CN)
• DONG, Qi
  Shijiazhuang, Hebei 050035 (CN)
• WANG, Dong
  Shijiazhuang, Hebei 050035 (CN)
• XUE, Jianfei
  Shijiazhuang, Hebei 050035 (CN)

(74) Representative: Gille Hrabal
Partnerschaftsgesellschaft mbB
Patentanwälte
Brucknerstraße 20
40593 Düsseldorf (DE)

(54) **USE OF MITOXANTRONE LIPOSOME IN COMBINATION WITH CAPECITABINE IN TREATING NASOPHARYNGEAL CARCINOMA**

(57) Use of a mitoxantrone liposome and capecitabine in treating nasopharyngeal carcinoma, in particular recurrent and/or metastatic nasopharyngeal carcinoma. The present invention further provides a method for treating nasopharyngeal carcinoma, in particular recurrent and/or metastatic nasopharyngeal carcinoma, the method comprising administering a therapeutically effective dose of the mitoxantrone liposome and capecitabine to a patient with nasopharyngeal carcinoma. The combination of the mitoxantrone liposome and capecitabine exhibits superior efficacy in patients with nasopharyngeal carcinoma, thereby providing a new treatment option for patients with nasopharyngeal carcinoma.

EP 4 582 088 A1

**Description**

**TECHNICAL FIELD**

The present disclosure pertains to the anti-tumor field and particularly relates to use of a mitoxantrone liposome and capecitabine for the treatment of nasopharyngeal carcinoma, especially of recurrent and/or metastatic nasopharyngeal carcinoma.

**BACKGROUND**

Nasopharyngeal carcinoma (NPC) is a common malignant tumor of the head and neck, originating from the nasopharyngeal mucosa. It is commonly found in the pharyngeal recess and exhibits significant geographic prevalence. NPC is prevalent in Southern China, Southeast Asia, and North Africa, where the age-standardized incidence is 4-25 cases/100,000 people, which is 50-100 times higher than in other regions of the world. According to the GLOBOCAN data published by the International Agency for Research on Cancer in 2020, there were about 133,354 new cases worldwide, and over 70% of the cases concentrated in these high-incidence regions. The prognosis for early-stage nasopharyngeal carcinoma is relatively good, with a 5-year survival rate of 80%-95%. However, the 5-year survival rate is only 40%-50% for patients with advanced-stage NPC. About 30% of patients with nasopharyngeal carcinoma experience recurrence and/or metastasis after initial treatment. Chemotherapy with platinum-based drugs combined with gemcitabine as a standard first-line treatment for inoperable recurrent or metastatic nasopharyngeal carcinoma can prolong the survival of patients, with a median overall survival (OS) of about 20 months (Ma SX, Zhou T, Huang Y, et al. The efficacy of first line chemotherapy in recurrent or metastatic nasopharyngeal carcinoma: a systematic review and meta analysis. Ann Transl Med, 2018, 6(11):201).

For patients with recurrent nasopharyngeal carcinoma, only a small fraction may receive radical local treatments again, such as surgery or radiotherapy. Most patients with recurrent NPC require palliative systemic treatment or best supportive care as do patients with metastatic NPC. The current standard first-line treatment regimens for recurrent or metastatic nasopharyngeal carcinoma are PD-1 inhibitors combined with gemcitabine and cisplatin. For patients who have failed first-line platinum-based therapy, few treatment regimens are available for patients with recurrent nasopharyngeal carcinoma who experience disease progression after first-line chemotherapy, and standard rescue treatment regimens are lacking. These patients are more encouraged to participate in clinical trials, or they generally receive monotherapies using drugs unused in first-line therapy, including gemcitabine, docetaxel, capecitabine, toripalimab, etc.

In summary, the treatment of recurrent and/or metastatic nasopharyngeal carcinoma is thorny, and especially for patients who have failed first-line platinum-based therapy, the efficacy of therapeutic drugs (including chemotherapy, immunotherapy, etc.) is poor, and the choices are limited. Therefore, there is an urgent need to explore new drugs or therapies for the treatment of recurrent and/or metastatic nasopharyngeal carcinoma.

Mitoxantrone is an anthracycline drug and has been used to date in over 30 countries worldwide. Mitoxantrone has a therapeutic effect on blood system tumors such as acute leukemia, lymphoma, and many solid tumors such as breast cancer, and the adverse effects are mainly myelosuppression, gastrointestinal reactions, and cardiotoxicity. Liposomes are a novel form for loading drugs. Studies have shown that they can alter the in vivo distribution of encapsulated drugs, allowing the drugs to accumulate primarily in tumor tissues, thereby increasing the therapeutic index of the drugs, reducing the therapeutic dose of the drugs, and lowing the toxicity of the drugs. Due to these properties, the use of liposome-loaded drugs in the research of anti-tumor drugs is highly valued. Studies have been conducted by researchers on mitoxantrone liposome formulations; for example, WO2008/080367A1 discloses a mitoxantrone liposome (the disclosure of which is incorporated herein by reference in its entirety) that has a better anti-tumor effect and reduced toxicity compared to free mitoxantrone. Based on these advantageous properties of the mitoxantrone liposome, it is expected to show beneficial effects in the treatment of more tumor types.

**SUMMARY**

The object of the present disclosure is to provide a new drug and a new therapy for the treatment of nasopharyngeal carcinoma, particularly of recurrent and/or metastatic nasopharyngeal carcinoma, which can improve the response rate and prolong survival, and have favorable safety and tolerability.

The present disclosure relates to use of a mitoxantrone liposome in combination with capecitabine for the treatment of nasopharyngeal carcinoma.

A first aspect of the present disclosure relates to use of a mitoxantrone liposome and capecitabine for manufacturing a medicament for the treatment of nasopharyngeal carcinoma.

In some embodiments, the nasopharyngeal carcinoma is recurrent and/or metastatic nasopharyngeal carcinoma. In some embodiments, the nasopharyngeal carcinoma is recurrent and/or metastatic nasopharyngeal carcinoma that has failed prior first-line or second-line platinum-based therapy.

In some embodiments, the mitoxantrone liposome and capecitabine are present in a same formulation. In some embodiments, the mitoxantrone liposome and capecitabine are each present in separate formulations. The formulation may be in any clinically acceptable dosage form, such as an injection dosage form, an oral dosage form, and the like. The injection dosage form includes a liquid injection, a powder for injection, a tablet for injection, and the like; the oral dosage form includes a tablet, a capsule, an oral liquid formulation, and the like.

In some embodiments, the mitoxantrone liposome is an injection, including a liquid injection, a powder for injection, a tablet for injection, and the like. When the mitoxantrone liposome is a liquid injection, the liposome comprises an active ingredient at 0.5-5 mg/mL, preferably 1-2 mg/mL, and more preferably 1 mg/mL, on the basis of mitoxantrone.

In some embodiments, capecitabine is a tablet with a strength of 0.15 g/tablet or 0.5 g/tablet, wherein the tablet with a strength of 0.15 g/tablet or 0.5 g/tablet means that: each tablet comprises 0.15 g or 0.5 g of capecitabine.

In some embodiments, use of the mitoxantrone liposome and capecitabine for manufacturing a medicament for the treatment of recurrent and/or metastatic nasopharyngeal carcinoma is provided. In some embodiments, use of the mitoxantrone liposome and capecitabine for manufacturing a medicament for the treatment of recurrent and/or metastatic nasopharyngeal carcinoma that has failed prior first-line or second-line platinum-based therapy. In these embodiments, the mitoxantrone liposome is an injection comprising an active ingredient at 0.5-5 mg/mL, on the basis of mitoxantrone; the capecitabine is a tablet with a strength of 0.15 g/tablet or 0.5 g/tablet.

A second aspect of the present disclosure relates to use of a mitoxantrone liposome for manufacturing a medicament for improving efficacy of capecitabine in treating nasopharyngeal carcinoma.

In some embodiments, the nasopharyngeal carcinoma is recurrent and/or metastatic nasopharyngeal carcinoma. In some embodiments, the nasopharyngeal carcinoma is recurrent and/or metastatic nasopharyngeal carcinoma that has failed prior first-line or second-line platinum-based therapy.

In some embodiments, the mitoxantrone liposome is an injection, including a liquid injection, a powder for injection, a tablet for injection, and the like. When the mitoxantrone liposome is a liquid injection, the liposome comprises, an active ingredient at 0.5-5 mg/mL, preferably 1-2 mg/mL, and more preferably 1 mg/mL, on the basis of mitoxantrone.

In some embodiments, capecitabine is a tablet with a strength of 0.15 g/tablet or 0.5 g/tablet, wherein the tablet with a strength of 0.15 g/tablet or 0.5 g/tablet means that: each tablet comprises 0.15 g or 0.5 g of capecitabine.

In some embodiments, use of the mitoxantrone liposome for manufacturing a medicament for improving efficacy of capecitabine in treating recurrent and/or metastatic nasopharyngeal carcinoma is provided. In some embodiments, use of the mitoxantrone liposome for manufacturing a medicament for improving efficacy of capecitabine in treating recurrent and/or metastatic nasopharyngeal carcinoma that has failed prior first-line or second-line platinum-based therapy is provided. In these embodiments, the mitoxantrone liposome is an injection comprising an active ingredient at 0.5-5 mg/mL, on the basis of mitoxantrone; the capecitabine is a tablet with a strength of 0.15 g/tablet or 0.5 g/tablet.

A third aspect of the present disclosure relates to use of capecitabine for manufacturing a medicament for improving efficacy of a mitoxantrone liposome in treating nasopharyngeal carcinoma.

In some embodiments, the nasopharyngeal carcinoma is recurrent and/or metastatic nasopharyngeal carcinoma. In some embodiments, the nasopharyngeal carcinoma is recurrent and/or metastatic nasopharyngeal carcinoma that has failed prior first-line or second-line platinum-based therapy.

In some embodiments, the mitoxantrone liposome is an injection, including a liquid injection, a powder for injection, a tablet for injection, and the like. When the mitoxantrone liposome is a liquid injection, the liposome comprises an active ingredient at 0.5-5 mg/mL, preferably 1-2 mg/mL, and more preferably 1 mg/mL, on the basis of mitoxantrone.

In some embodiments, capecitabine is a tablet with a strength of 0.15 g/tablet or 0.5 g/tablet, wherein the tablet with a strength of 0.15 g/tablet or 0.5 g/tablet means that: each tablet comprises 0.15 g or 0.5 g of capecitabine.

In some embodiments, use of capecitabine for manufacturing a medicament for improving efficacy of the mitoxantrone liposome in treating recurrent and/or metastatic nasopharyngeal carcinoma is provided. In some embodiments, use of capecitabine for manufacturing a medicament for improving efficacy of the mitoxantrone liposome in treating recurrent and/or metastatic nasopharyngeal carcinoma that has failed prior first-line or second-line platinum-based therapy is provided. In these embodiments, the mitoxantrone liposome is an injection comprising an active ingredient at 0.5-5 mg/mL, on the basis of mitoxantrone; the capecitabine is a tablet with a strength of 0.15 g/tablet or 0.5 g/tablet.

A fourth aspect of the present disclosure relates to a method of treatment of nasopharyngeal carcinoma, wherein the method comprises administering to a patient with nasopharyngeal carcinoma a therapeutically effective amount of a mitoxantrone liposome and capecitabine.

A fifth aspect of the present disclosure relates to a method for improving efficacy of capecitabine in treating nasopharyngeal carcinoma, wherein the method comprises administering capecitabine to a patient with nasopharyngeal carcinoma in combination with a therapeutically effective amount of a mitoxantrone liposome.

A sixth aspect of the present disclosure relates to a method for improving efficacy of a mitoxantrone liposome in treating nasopharyngeal carcinoma, wherein the method comprises administering the mitoxantrone liposome to a patient with nasopharyngeal carcinoma in combination with a therapeutically effective amount of capecitabine.

The above fourth to sixth aspects comprise the following embodiments.

In some embodiments, the nasopharyngeal carcinoma is recurrent and/or metastatic nasopharyngeal carcinoma. In some embodiments, the nasopharyngeal carcinoma is recurrent and/or metastatic nasopharyngeal carcinoma that has failed prior first-line or second-line platinum-based therapy.

In some embodiments, the mitoxantrone liposome is administered by injection. In some embodiments, the mitoxantrone liposome is administered by injection at a therapeutically effective dose of 8-30 mg/m$^2$, preferably 12-24 mg/m$^2$, and more preferably 12-20 mg/m$^2$. In some embodiments, the mitoxantrone liposome is administered on day 1 of each administration cycle. In some embodiments, the mitoxantrone liposome is administered by injection at a therapeutically effective dose of 8-30 mg/m$^2$ on day 1 of each administration cycle. In some embodiments, the mitoxantrone liposome is administered by injection at a therapeutically effective dose of, for example, 12 mg/m$^2$, 14 mg/m$^2$, 15 mg/m$^2$, 16 mg/m$^2$, 18 mg/m$^2$, 20 mg/m$^2$, or 24 mg/m$^2$ on day 1 of each administration cycle. Preferably, the administration cycle is 3 weeks. Preferably, the mitoxantrone liposome is administered by intravenous drip for 30 min-120 min; preferably 60 min-120 min. In some embodiments, capecitabine is administered orally. In some embodiments, capecitabine is administered orally at the dose of 500 mg/m$^2$, 750 mg/m$^2$, or 1000 mg/m$^2$. In some embodiments, capecitabine is administered twice daily. In some embodiments, capecitabine is administered on days 1-14 of each administration cycle. In some embodiments, capecitabine is administered in a tablet form. In some embodiments, capecitabine is administered orally at the dose of 500 mg/m$^2$, 750 mg/m$^2$, or 1000 mg/m$^2$ twice daily on days 1-14 of each administration cycle. In some embodiments, capecitabine is administered orally in a tablet form at the dose of 1000 mg/m$^2$ twice daily on days 1-14 of each administration cycle. Preferably, the administration cycle is 3 weeks.

In some embodiments, during each administration cycle, the mitoxantrone liposome is administered once, and the capecitabine is administered for 14 consecutive days. Preferably, a therapeutically effective dose of the mitoxantrone liposome and capecitabine are administered sequentially in any order to a patient with nasopharyngeal carcinoma on day 1 of each administration cycle, and then capecitabine continues to be administered twice daily on days 2-14. In some embodiments, the mitoxantrone liposome is administered by injection at a therapeutically effective dose of 8-30 mg/m$^2$ and capecitabine is administered orally at the dose of 500 mg/m$^2$, 750 mg/m$^2$, or 1000 mg/m$^2$ on day 1 of each administration cycle, and then capecitabine continues to be administered orally at the dose of 500 mg/m$^2$, 750 mg/m$^2$, or 1000 mg/m$^2$ on days 2-14 of the cycle. In some embodiments, the mitoxantrone liposome is administered by injection at a therapeutically effective dose of 12-24 mg/m$^2$ once and capecitabine is administered orally at the dose of 1000 mg/m$^2$ twice on day 1 of each administration cycle, and then capecitabine continues to be administered orally at the dose of 1000 mg/m$^2$ twice daily on days 2-14 of the cycle.

A seventh aspect of the present disclosure relates to a medicament for the treatment of nasopharyngeal carcinoma, wherein the medicament comprises a mitoxantrone liposome and capecitabine.

In some embodiments, the nasopharyngeal carcinoma is recurrent and/or metastatic nasopharyngeal carcinoma. In some embodiments, the nasopharyngeal carcinoma is recurrent and/or metastatic nasopharyngeal carcinoma that has failed prior first-line or second-line platinum-based therapy.

In some embodiments, the mitoxantrone liposome and capecitabine are present in a same formulation. In some embodiments, the mitoxantrone liposome and capecitabine are each present in separate formulations. The formulation may be in any clinically acceptable dosage form, such as an injection dosage form, an oral dosage form, and the like. The injection dosage form includes a liquid injection, a powder for injection, a tablet for injection, and the like; the oral dosage form includes a tablet, a capsule, an oral liquid formulation, and the like.

In some embodiments, the mitoxantrone liposome is an injection, including a liquid injection, a powder for injection, a tablet for injection, and the like. When the mitoxantrone liposome is a liquid injection, the liposome comprises an active ingredient at 0.5-5 mg/mL, preferably 1-2 mg/mL, and more preferably 1 mg/mL, on the basis of mitoxantrone.

In some embodiments, capecitabine is a tablet with a strength of 0.15 g/tablet or 0.5 g/tablet, wherein the tablet with a strength of 0.15 g/tablet or 0.5 g/tablet means that: each tablet comprises 0.15 g or 0.5 g of capecitabine.

In some embodiments, the medicament may further comprise other medicaments for the treatment of nasopharyngeal carcinoma, including medicaments approved for the treatment of nasopharyngeal carcinoma by drug administrations of China or other countries and regions (for example, the United States of America, the European Union, Japan, the Republic of Korea, etc.).

In some embodiments, the mitoxantrone liposome is administered by injection. In some embodiments, the mitoxantrone liposome is administered by injection at a therapeutically effective amount of 8-30 mg/m$^2$, preferably 12-24 mg/m$^2$, and more preferably 12-20 mg/m$^2$. In some embodiments, the mitoxantrone liposome is administered on day 1 of each administration cycle. In some embodiments, the mitoxantrone liposome is administered by injection at a therapeutically effective amount of 8-30 mg/m$^2$ on day 1 of each administration cycle. In some embodiments, the mitoxantrone liposome is administered by injection at a therapeutically effective amount of, for example, 12 mg/m$^2$, 14 mg/m$^2$, 15 mg/m$^2$, 16 mg/m$^2$, 18 mg/m$^2$, 20 mg/m$^2$, or 24 mg/m$^2$ on day 1 of each administration cycle. Preferably, the administration cycle is 3 weeks. Preferably, the mitoxantrone liposome is administered by intravenous drip for 30 min-120 min; preferably, the mitoxantrone liposome is administered by intravenous drip for 60 min-120 min.

In some embodiments, capecitabine is administered orally. In some embodiments, capecitabine is administered orally at a dose of 500 mg/m$^2$, 750 mg/m$^2$, or 1000 mg/m$^2$. In some embodiments, capecitabine is administered twice daily. In some embodiments, capecitabine is administered on days 1-14 of each administration cycle. In some embodiments, capecitabine is administered in a tablet form. In some embodiments, capecitabine is administered orally at a dose of 500 mg/m$^2$, 750 mg/m$^2$, or 1000 mg/m$^2$ twice daily on days 1-14 of each administration cycle. In some embodiments, capecitabine is administered orally in a tablet form at a dose of 1000 mg/m$^2$ twice daily on days 1-14 of each administration cycle. Preferably, the administration cycle is 3 weeks.

In some embodiments, during each administration cycle, the mitoxantrone liposome is administered once, and the capecitabine is administered for 14 consecutive days. Preferably, a therapeutically effective amount of the mitoxantrone liposome and capecitabine are administered sequentially in any order to a patient with nasopharyngeal carcinoma on day 1 of each administration cycle, and then capecitabine continues to be administered twice daily on days 2-14. In some embodiments, the mitoxantrone liposome is administered by injection at a therapeutically effective amount of 8-30 mg/m$^2$ and capecitabine is administered orally at a dose of 500 mg/m$^2$, 750 mg/m$^2$, or 1000 mg/m$^2$ on day 1 of each administration cycle, and then capecitabine continues to be administered orally at a dose of 500 mg/m$^2$, 750 mg/m$^2$, or 1000 mg/m$^2$ on days 2-14 of the cycle. In some embodiments, the mitoxantrone liposome is administered by injection at a therapeutically effective amount of 12-24 mg/m$^2$ once and capecitabine is administered orally at a dose of 1000 mg/m$^2$ twice on day 1 of each administration cycle, and then capecitabine continues to be administered orally at a dose of 1000 mg/m$^2$ twice daily on days 2-14 of the cycle.

An eighth aspect of the present disclosure relates to a medicament for improving efficacy of capecitabine in treating nasopharyngeal carcinoma, wherein the medicament comprises a mitoxantrone liposome.

A ninth aspect of the present disclosure relates to a medicament for improving efficacy of a mitoxantrone liposome in treating nasopharyngeal carcinoma, wherein the medicament comprises capecitabine.

The above eighth and ninth aspects comprise the following embodiments.

In some embodiments, the nasopharyngeal carcinoma is recurrent and/or metastatic nasopharyngeal carcinoma. In some embodiments, the nasopharyngeal carcinoma is recurrent and/or metastatic nasopharyngeal carcinoma that has failed prior first-line or second-line platinum-based therapy.

In some embodiments, the mitoxantrone liposome is an injection, including a liquid injection, a powder for injection, a tablet for injection, and the like. When the mitoxantrone liposome is a liquid injection, the liposome comprises an active ingredient at 0.5-5 mg/mL, preferably 1-2 mg/mL, and more preferably 1 mg/mL, on the basis of mitoxantrone.

In some embodiments, capecitabine is a tablet with a strength of 0.15 g/tablet or 0.5 g/tablet, wherein the tablet with a strength of 0.15 g/tablet or 0.5 g/tablet means that: each tablet comprises 0.15 g or 0.5 g of capecitabine.

In some embodiments, the mitoxantrone liposome is administered by injection. In some embodiments, the mitoxantrone liposome is administered by injection at a therapeutically effective amount of 8-30 mg/m$^2$, preferably 12-24 mg/m$^2$, and more preferably 12-20 mg/m$^2$. In some embodiments, the mitoxantrone liposome is administered on day 1 of each administration cycle. In some embodiments, the mitoxantrone liposome is administered by injection at a therapeutically effective amount of 8-30 mg/m$^2$ on day 1 of each administration cycle. In some embodiments, the mitoxantrone liposome is administered by injection at a therapeutically effective amount of, for example, 12 mg/m$^2$, 14 mg/m$^2$, 15 mg/m$^2$, 16 mg/m$^2$, 18 mg/m$^2$, 20 mg/m$^2$, or 24 mg/m$^2$ on day 1 of each administration cycle. Preferably, the administration cycle is 3 weeks. Preferably, the mitoxantrone liposome is administered by intravenous drip for 30 min-120 min; preferably, the mitoxantrone liposome is administered by intravenous drip for 60 min-120 min.

In some embodiments, capecitabine is administered orally. In some embodiments, capecitabine is administered orally at a dose of 500 mg/m$^2$, 750 mg/m$^2$, or 1000 mg/m$^2$. In some embodiments, capecitabine is administered twice daily. In some embodiments, capecitabine is administered on days 1-14 of each administration cycle. In some embodiments, capecitabine is administered in a tablet form. In some embodiments, capecitabine is administered orally at a dose of 500 mg/m$^2$, 750 mg/m$^2$, or 1000 mg/m$^2$ twice daily on days 1-14 of each administration cycle. In some embodiments, capecitabine is administered orally in a tablet form at a dose of 1000 mg/m$^2$ twice daily on days 1-14 of each administration cycle. Preferably, the administration cycle is 3 weeks.

In some embodiments, during each administration cycle, the mitoxantrone liposome is administered once, and the capecitabine is administered for 14 consecutive days. Preferably, a therapeutically effective amount of the mitoxantrone liposome and capecitabine are administered sequentially in any order to a patient with nasopharyngeal carcinoma on day 1 of each administration cycle, and then capecitabine continues to be administered twice daily on days 2-14. In some embodiments, the mitoxantrone liposome is administered by injection at a therapeutically effective amount of 8-30 mg/m$^2$ and capecitabine is administered orally at a dose of 500 mg/m$^2$, 750 mg/m$^2$, or 1000 mg/m$^2$ on day 1 of each administration cycle, and then capecitabine continues to be administered orally at a dose of 500 mg/m$^2$, 750 mg/m$^2$, or 1000 mg/m$^2$ on days 2-14 of the cycle. In some embodiments, the mitoxantrone liposome is administered by injection at a therapeutically effective amount of 12-24 mg/m$^2$ once and capecitabine is administered orally at a dose of 1000 mg/m$^2$ twice on day 1 of each administration cycle, and then capecitabine continues to be administered orally at a dose of 1000 mg/m$^2$ twice daily on days 2-14 of the cycle.

A tenth aspect of the present disclosure relates to a composition for the treatment of nasopharyngeal carcinoma, wherein the composition comprises a mitoxantrone liposome and capecitabine.

An eleventh aspect of the present disclosure relates to a pharmaceutical combination for the treatment of nasopharyngeal carcinoma, wherein the pharmaceutical combination comprises a mitoxantrone liposome and capecitabine. The above tenth and eleventh aspects comprise the following embodiments.

In some embodiments, the nasopharyngeal carcinoma is recurrent and/or metastatic nasopharyngeal carcinoma. In some embodiments, the nasopharyngeal carcinoma is recurrent and/or metastatic nasopharyngeal carcinoma that has failed prior first-line or second-line platinum-based therapy.

In some embodiments, the mitoxantrone liposome is an injection, including a liquid injection, a powder for injection, a tablet for injection, and the like. When the mitoxantrone liposome is a liquid injection, the liposome comprises an active ingredient at 0.5-5 mg/mL, preferably 1-2 mg/mL, and more preferably 1 mg/mL, on the basis of mitoxantrone.

In some embodiments, capecitabine is a tablet with a strength of 0.15 g/tablet or 0.5 g/tablet, wherein the tablet with a strength of 0.15 g/tablet or 0.5 g/tablet means that: each tablet comprises 0.15 g or 0.5 g of capecitabine.

In some embodiments, the mitoxantrone liposome is administered by injection. In some embodiments, the mitoxantrone liposome is administered by injection at a therapeutically effective amount of 8-30 mg/m$^2$, preferably 12-24 mg/m$^2$, and more preferably 12-20 mg/m$^2$. In some embodiments, the mitoxantrone liposome is administered on day 1 of each administration cycle. In some embodiments, the mitoxantrone liposome is administered by injection at a therapeutically effective amount of 8-30 mg/m$^2$ on day 1 of each administration cycle. In some embodiments, the mitoxantrone liposome is administered by injection at a therapeutically effective amount of, for example, 12 mg/m$^2$, 14 mg/m$^2$, 15 mg/m$^2$, 16 mg/m$^2$, 18 mg/m$^2$, 20 mg/m$^2$, or 24 mg/m$^2$ on day 1 of each administration cycle. Preferably, the administration cycle is 3 weeks. Preferably, the mitoxantrone liposome is administered by intravenous drip for 30 min-120 min; preferably, the mitoxantrone liposome is administered by intravenous drip for 60 min-120 min.

In some embodiments, capecitabine is administered orally. In some embodiments, capecitabine is administered orally at a dose of 500 mg/m$^2$, 750 mg/m$^2$, or 1000 mg/m$^2$. In some embodiments, capecitabine is administered twice daily. In some embodiments, capecitabine is administered on days 1-14 of each administration cycle. In some embodiments, capecitabine is administered in a tablet form. In some embodiments, capecitabine is administered orally at a dose of 500 mg/m$^2$, 750 mg/m$^2$, or 1000 mg/m$^2$ twice daily on days 1-14 of each administration cycle. In some embodiments, capecitabine is administered orally in a tablet form at a dose of 1000 mg/m$^2$ twice daily on days 1-14 of each administration cycle. Preferably, the administration cycle is 3 weeks.

In some embodiments, during each administration cycle, the mitoxantrone liposome is administered once, and the capecitabine is administered for 14 consecutive days. Preferably, a therapeutically effective amount of the mitoxantrone liposome and capecitabine are administered sequentially in any order to a patient with nasopharyngeal carcinoma on day 1 of each administration cycle, and then capecitabine continues to be administered twice daily on days 2-14. In some embodiments, the mitoxantrone liposome is administered by injection at a therapeutically effective amount of 8-30 mg/m$^2$ and capecitabine is administered orally at a dose of 500 mg/m$^2$, 750 mg/m$^2$, or 1000 mg/m$^2$ on day 1 of each administration cycle, and then capecitabine continues to be administered orally at a dose of 500 mg/m$^2$, 750 mg/m$^2$, or 1000 mg/m$^2$ on days 2-14 of the cycle. In some embodiments, the mitoxantrone liposome is administered by injection at a therapeutically effective amount of 12-24 mg/m$^2$ once and capecitabine is administered orally at a dose of 1000 mg/m$^2$ twice on day 1 of each administration cycle, and then capecitabine continues to be administered orally at a dose of 1000 mg/m$^2$ twice daily on days 2-14 of the cycle.

A twelfth aspect of the present disclosure relates to a kit for the treatment of nasopharyngeal carcinoma, wherein the kit comprises a mitoxantrone liposome and capecitabine.

In some embodiments, the nasopharyngeal carcinoma is recurrent and/or metastatic nasopharyngeal carcinoma. In some embodiments, the nasopharyngeal carcinoma is recurrent and/or metastatic nasopharyngeal carcinoma that has failed prior first-line or second-line platinum-based therapy.

In some embodiments, the mitoxantrone liposome and capecitabine are each separate formulations.

In some embodiments, the mitoxantrone liposome is an injection, including a liquid injection, a powder for injection, a tablet for injection, and the like. When the mitoxantrone liposome is a liquid injection, the liposome comprises, an active ingredient at 0.5-5 mg/mL, preferably 1-2 mg/mL, and more preferably 1 mg/mL, on the basis of mitoxantrone.

In some embodiments, capecitabine is a tablet with a strength of 0.15 g/tablet or 0.5 g/tablet, wherein the tablet with a strength of 0.15 g/tablet or 0.5 g/tablet means that: each tablet comprises 0.15 g or 0.5 g of capecitabine.

In the above aspects, capecitabine may be provided in a form of a pharmaceutical formulation, such as a tablet. Such pharmaceutical formulations are commercially available.

In the above aspects, "mitoxantrone" comprises mitoxantrone and pharmaceutically acceptable salts thereof, preferably mitoxantrone hydrochloride.

The mitoxantrone liposome is preferably a mitoxantrone hydrochloride liposome.

In the above aspects, the mitoxantrone liposome is not particularly limited. Without being bound by any particular theory, the inventors of the present application have discovered that one or more of the following properties are advantageous to the mitoxantrone liposome formulation:

(i) the mitoxantrone liposome is a mitoxantrone hydrochloride liposome;
(ii) the mitoxantrone liposome has a particle size of about 30-80 nm, for example, about 35-75 nm, about 40-70 nm, about 40-60 nm, or about 60 nm, for example, about 30, 35, 40, 45, 50, 55, 60, 65, 70, 75, or 80 nm; there are various methods of particle size determination, including but not limited to NanoZS;
(iii) mitoxantrone forms a poorly soluble precipitate with multivalent counterions(e.g., sulfate anion, citrate anion, or phosphate anion) in the liposome;
(iv) a phospholipid bilayer in the mitoxantrone liposome comprises a phospholipid with a phase transition temperature (Tm) higher than body temperature, such that the liposome has a phase transition temperature higher than body temperature; the phospholipid includes, but is not limited to hydrogenated soybean lecithin, phosphatidyl choline, hydrogenated yolk lecithin, dipalmitoyl lecithin, distearoyl lecithin, or any combination thereof;
(v) a phospholipid bilayer in the mitoxantrone liposome comprises hydrogenated soybean lecithin, cholesterol, and polyethylene glycol 2000-modified distearoyl phosphatidyl ethanolamine (DSPE-PEG2000);
(vi) a phospholipid bilayer in the mitoxantrone liposome comprises hydrogenated soybean lecithin, cholesterol, and polyethylene glycol 2000-modified distearoyl phosphatidyl ethanolamine in a mass ratio of about 3:1:1; mitoxantrone hydrochloride forms a poorly soluble precipitate with multivalent counterions in the liposome, and the mitoxantrone hydrochloride liposome has a particle size of about 60 nm;
(vii) a mitoxantrone liposome formulation manufactured using a method disclosed in Chinese Patent Application No. 200610102339.8 or PCT Application No. WO2008/080367A1; and
(viii) the mitoxantrone liposome is a mitoxantrone liposome of NMPA Approval No. H20220001.

Preferably, the mitoxantrone liposome has one or more of the following properties:

(i) the mitoxantrone liposome is a mitoxantrone hydrochloride liposome;
(ii) the mitoxantrone liposome has a particle size of 30-80 nm;
(iii) mitoxantrone forms a poorly soluble precipitate with multivalent counterions in the liposome; and
(iv) a phospholipid bilayer in the mitoxantrone liposome comprises a phospholipid with a phase transition temperature (Tm) higher than body temperature, and the phospholipid is selected from hydrogenated soybean lecithin, phosphatidyl choline, hydrogenated yolk lecithin, dipalmitoyl lecithin, distearoyl lecithin, and any combination thereof.

Preferably, the mitoxantrone liposome is a mitoxantrone hydrochloride liposome. The therapeutically effective amount or dose of the mitoxantrone liposome or mitoxantrone hydrochloride liposome is calculated on the basis of mitoxantrone.

The mitoxantrone liposome may be manufactured by conventional methods in the art, or by any one of the methods disclosed in the prior art, such as the method disclosed in WO2008/080367 A1, which is incorporated herein by reference in its entirety. As a non-limiting example, the mitoxantrone hydrochloride liposome formulation of the present application may be manufactured according to the method below, wherein the "lipid-to-drug ratio" refers to the mass ratio of the constituents of the phospholipid bilayer (comprising HSPC, DSPE-PEG2000, and Chol) to mitoxantrone in the liposome:

Hydrogenated soybean lecithin (HSPC), cholesterol (Chol), and polyethylene glycol 2000-modified distearoyl phosphatidyl ethanolamine (DSPE-PEG2000) are weighed out according to a mass ratio of 3:1:1 and dissolved in 95% ethanol to obtain a clear solution (namely, a solution of phospholipids in ethanol). The solution of phospholipids in ethanol is mixed with a 300 mM ammonium sulfate solution, and the resulting mixture is shaken for hydration at 60-65 °C for 1 h to obtain a heterogeneous multivesicular liposome. Then the granularity of the liposome is reduced using a microfluidizer. The obtained sample is 200-fold diluted with a NaCl solution having a concentration of 0.9% and then analyzed with NanoZS. The average granularity of the particles is about 60 nm, and the main peak is between 40-60 nm. The ammonium sulfate in the external phase of the blank liposome is then removed using an ultrafiltration device, and the external phase is replaced with 290 mM sucrose and 10 mM glycine in order to form a transmembrane ammonium sulfate gradient. A mitoxantrone hydrochloride solution (10 mg/mL on the basis of mitoxantron) is added to the blank liposome in a lipid-to-drug ratio of 16:1, and drug loading is performed at 60-65°C. After about 1 h of incubation, the encapsulation efficiency can be shown to be about 100% using gel size exclusion chromatography. The product obtained is named PLM60. PLM60 contains HSPC, Chol, DSPE-PEG2000, and mitoxantrone in a weight ratio of 9.58:3.19:3.19:1, and the osmotic pressure of the sucrose-glycine solution is close to the physiological value.

It should be understood that a plurality of technical details and parameters of the exemplary preparation method described above can be tuned and determined within a reasonable scope by those skilled in the art. For example, amino acid alternatives to the glycine in the external phase used to form the transmembrane ammonium sulfate gradient include, but are not limited to, histidine, asparagine, glutamic acid, leucine, proline, and alanine. As another example, the mass ratio of HSPC, Chol, and DSPE-PEG2000 can be appropriately adjusted. As another example, regarding the lipid-to-drug ratio parameter in manufacturing a particular liposome pharmaceutical formulation, one skilled in the art can design, test, and ultimately arrive at an appropriate lipid-to-drug ratio to maximize the drug-loading rate while reducing drug leakage. For the mitoxantrone hydrochloride liposome formulation of the present application, a wide range of lipid-to-drug ratios can be used, for example, as low as 2:1 or as high as 30:1, 40:1, or 50:1, and a more suitable lipid-to-drug ratio can be about (15-20):1, for example, about 15:1, 16:1, 17:1, 18:1, 19:1, or 20:1. Thus, the several advantageous properties of the mitoxantrone hydrochloride liposome formulation described above are more important, and the methodologies for achieving these properties are varied.

In the context of the present disclosure, unless defined otherwise, all technical and scientific terms used have the same meaning as commonly understood by those of ordinary skill in the art.

The term "contain" (or its equivalents such as "comprise", "include", etc.) as used herein includes the case of "consist of...", unless otherwise specified.

The term "composition" as used herein means a combination of two or more active ingredients, wherein the active ingredients can be present in the same formulation so as to be administered together, or in different formulations and are administered simultaneously or sequentially using the same route of administration or different routes of administration. In this sense, "composition" and "combination" are interchangeable.

The term "therapeutically effective amount" or "effective amount" as used herein refers to a dose that shows an expected benefit in the treated subject. The "therapeutically effective amount" or the "effective amount" will depend upon the species of the treated subject, the severity of the disease, the frequency of administration, the metabolic profile of the drug, and other factors, and can be determined by the prescribing physician in accordance with routine practice. It should be noted that each one of all numerical ranges mentioned herein includes the two endpoints of the range, all numbers within the range, and sub-ranges delimited by any two of the numbers.

The term "treatment" as used herein refers to controlling, alleviating, or ameliorating the pathological progression of a disease and prolonging the survival of a subject with the disease.

The terms "subject" and "patient" as used herein include animal individuals to which the drugs or compositions of the present application are expected to be administered, including but not limited to humans and other mammals, such as mice, rats, cats, monkeys, dogs, horses, pigs, etc. Preferably, the subject is a human. The terms "subject" and "patient" are interchangeable unless indicated otherwise.

The term "recurrent nasopharyngeal carcinoma" as used herein refers to the lesions confirmed by pathological diagnosis as nasopharyngeal carcinoma has completely disappears after radical radiotherapy, and a lesion reappears locally or regionally with the same pathological type as the original tumor 6 months after the completion of treatment.

The term "metastatic nasopharyngeal carcinoma" as used herein refers to nasopharyngeal carcinoma with distant metastasis.

The term "administered sequentially in any order" as used herein means that separate formulations of the mitoxantrone liposome and capecitabine are administered separately in a clinically acceptable manner, without any strict requirements regarding the order of administration, and the drugs are not mixed in vitro.

*Other Embodiments Of the Present Disclosure*

Embodiment 1. Use of a mitoxantrone liposome and capecitabine for manufacturing a medicament for the treatment of nasopharyngeal carcinoma, wherein preferably, the nasopharyngeal carcinoma is recurrent/metastatic nasopharyngeal carcinoma; preferably, the recurrent/metastatic nasopharyngeal carcinoma has failed prior first-line or second-line platinum-based therapy; more preferably, the recurrent/metastatic nasopharyngeal carcinoma has at least one evaluable lesion at baseline according to the RECIST 1.1 criteria, and the area should not have undergone radiotherapy, or the radiotherapy lesion has clear radiographic progressed after radiotherapy.

Embodiment 2. Use of a mitoxantrone liposome for manufacturing a medicament for improving efficacy of a capecitabine treatment regime in treating nasopharyngeal carcinoma, wherein preferably, the nasopharyngeal carcinoma is recurrent metastatic nasopharyngeal carcinoma; preferably, the recurrent metastatic nasopharyngeal carcinoma is recurrent metastatic nasopharyngeal carcinoma that has failed prior first-line or second-line platinum-based therapy; more preferably, the recurrent metastatic nasopharyngeal carcinoma has at least one assessable lesion at baseline according to the RECIST 1.1 criteria, and the area must not have undergone radiotherapy, or evidence indicates that the lesion exhibits significant progression after radiotherapy.

Embodiment 3. The use according to embodiment 1 or 2, wherein the mitoxantrone liposome is a mitoxantrone hydrochloride liposome.

Embodiment 4. The use according to embodiment 3, wherein the mitoxantrone hydrochloride liposome and capecitabine may be present in a same formulation or may be separately formulated; when the two drugs described above are separately formulated, dosage forms may be identical or different; when the mitoxantrone hydrochloride liposome is a liquid injection, the mitoxantrone hydrochloride liposome comprises an active ingredient at 0.5-5 mg/mL, preferably 1-2 mg/mL, and more preferably 1 mg/mL, on the basis of mitoxantrone; when capecitabine is a tablet, a strength is 0.15 g/tablet or 0.5 g/tablet.

Embodiment 5. The use according to any one of embodiments 1-4, wherein the medicament further comprises other medicaments for the treatment of nasopharyngeal carcinoma.

Embodiment 6. A medicament for the treatment of nasopharyngeal carcinoma, comprising a mitoxantrone liposome and capecitabine, wherein the mitoxantrone liposome is preferably a mitoxantrone hydrochloride liposome; the mitoxantrone hydrochloride liposome and capecitabine may be present in a same formulation or may be separately formulated; when the two drugs described above are separately formulated, dosage forms may be identical or different; when the mitoxantrone hydrochloride liposome is a liquid injection, the mitoxantrone hydrochloride liposome comprises an active ingredient at 0.5-5 mg/mL, preferably 1-2 mg/mL, and more preferably 1 mg/mL, on the basis of mitoxantrone; when capecitabine is a tablet, a strength is 0.15 g/tablet or 0.5 g/tablet.

Embodiment 7. The medicament according to embodiment 6, further comprising other medicaments for the treatment of nasopharyngeal carcinoma.

Embodiment 8. A method of treatment of nasopharyngeal carcinoma, wherein a therapeutically effective dose of a mitoxantrone hydrochloride liposome and capecitabine are administered to a patient with nasopharyngeal carcinoma; the mitoxantrone hydrochloride liposome is preferably administered by injection; capecitabine is preferably administered orally; preferably, the therapeutically effective dose of the mitoxantrone hydrochloride liposome refers to, 8-30 mg/m$^2$, more preferably 12-20 mg/m$^2$, on the basis of mitoxantrone; capecitabine is administered at 1000 mg/m$^2$ twice daily.

Embodiment 9. A method for improving efficacy of a capecitabine treatment regimen for nasopharyngeal carcinoma, wherein capecitabine is administered to a patient with nasopharyngeal carcinoma in combination with a therapeutically effective dose of a mitoxantrone hydrochloride liposome.

Embodiment 10. A composition for the treatment of nasopharyngeal carcinoma, comprising a mitoxantrone hydrochloride liposome and capecitabine, wherein a therapeutically effective amount of the mitoxantrone hydrochloride liposome and capecitabine are administered sequentially in any order to a patient with nasopharyngeal carcinoma on day 1 of each administration cycle, and capecitabine continues to be administered on days 2-14; preferably, the administration cycle is once every 3 weeks.

Embodiment 11. A medicament for improving efficacy of a capecitabine treatment regimen in treating nasopharyngeal carcinoma, comprising a mitoxantrone hydrochloride liposome, wherein the mitoxantrone hydrochloride liposome is administered at any time before or after capecitabine administration on day 1 of each administration cycle at a dose of 8-30 mg/m$^2$, more preferably 12-20 mg/m$^2$, once every 3 weeks.

Embodiment 12. The use according to any one of embodiments 1-5, wherein the mitoxantrone liposome has one or more of the following properties:

(i) the mitoxantrone hydrochloride liposome has a particle size of about 30-80 nm, for example, about 35-75 nm, about 40-70 nm, about 40-60 nm, or about 60 nm;
(ii) mitoxantrone hydrochloride forms a poorly soluble precipitate with multivalent counterions(e.g., sulfate anion , citrate anion, or phosphate anion) in the liposome;
(iii) a phospholipid bilayer in the mitoxantrone hydrochloride liposome comprises a phospholipid with a phase transition temperature (Tm) higher than body temperature, such that the liposome has a phase transition temperature higher than body temperature; for example, the phospholipid is selected from hydrogenated soybean lecithin, phosphatidyl choline, hydrogenated yolk lecithin, dipalmitoyl lecithin, distearoyl lecithin, and any combination thereof;
(iv) a phospholipid bilayer in the mitoxantrone hydrochloride liposome comprises hydrogenated soybean lecithin, cholesterol, and polyethylene glycol 2000-modified distearoyl phosphatidyl ethanolamine (DSPE-PEG2000); and
(v) a phospholipid bilayer in the mitoxantrone hydrochloride liposome comprises hydrogenated soybean lecithin, cholesterol, and polyethylene glycol 2000-modified distearoyl phosphatidyl ethanolamine in a mass ratio of about 3:1:1; mitoxantrone hydrochloride forms a poorly soluble precipitate with multivalent acid radicals in the liposome, and the mitoxantrone hydrochloride liposome has a particle size of about 60 nm in the medicament.

Embodiment 13. The medicament according to embodiment 6, 7, or 11, the method according to embodiment 8 or 9, or the composition according to claim 10, wherein the mitoxantrone liposome has one or more of the following properties:

(i) the mitoxantrone hydrochloride liposome has a particle size of about 30-80 nm, for example, about 35-75 nm, about 40-70 nm, about 40-60 nm, or about 60 nm;
(ii) mitoxantrone hydrochloride forms a poorly soluble precipitate with multivalent counterions(e.g., sulfate anion, citrate anion, or phosphate anion) in the liposome;
(iii) a phospholipid bilayer in the mitoxantrone hydrochloride liposome comprises a phospholipid with a phase transition temperature (Tm) higher than body temperature, such that the liposome has a phase transition temperature higher than body temperature; for example, the phospholipid is selected from hydrogenated soybean lecithin, phosphatidyl choline, hydrogenated yolk lecithin, dipalmitoyl lecithin, distearoyl lecithin, and any combination thereof; (iv) a phospholipid bilayer in the mitoxantrone hydrochloride liposome comprises hydrogenated soybean lecithin, cholesterol, and polyethylene glycol 2000-modified distearoyl phosphatidyl ethanolamine (DSPE-PEG2000); and (v) a phospholipid bilayer in the mitoxantrone hydrochloride liposome comprises hydrogenated soybean lecithin, cholesterol, and polyethylene glycol 2000-modified distearoyl phosphatidyl ethanolamine in a mass ratio of about 3:1:1; mitoxantrone hydrochloride forms a poorly soluble precipitate with multivalent acid radicals in the liposome, and the mitoxantrone hydrochloride liposome has a particle size of about 60 nm in the medicament.

Beneficial Effects

Compared to a capecitabine monotherapy regimen, the combination regimen of the mitoxantrone liposome of the present disclosure and capecitabine achieves a better best overall response in the treatment of recurrent and/or metastatic nasopharyngeal carcinoma and has favorable safety and tolerability profiles. Therefore, the combination regimen can serve as a new treatment option for patients with recurrent and/or metastatic nasopharyngeal carcinoma.

## DETAILED DESCRIPTION

The following examples are intended to specifically illustrate the present disclosure and should not be construed as limiting the scope of the present disclosure.

Example 1. A Randomized, Open-Label, Positive-Controlled, Multi-center Phase III Clinical Study of Mitoxantrone Hydrochloride Liposome Injection Combined with Capecitabine Versus Capecitabine Monotherapy in Subjects with Recurrent and/or Metastatic Nasopharyngeal Carcinoma Who Have Failed Platinum-Based Therapy.

The investigational drug, mitoxantrone hydrochloride liposome injection, was provided by CSPC Zhongnuo Pharmaceutical (Shijiazhuang) Co., Ltd. (NMPA Approval No. H20220001) at 1.0 mg/mL.

Capecitabine tablets were a commercially available formulation.

(I) Study objective

This study is a randomized, open-label, positive-controlled, multicenter phase III clinical study whose objective is to evaluate the efficacy and safety of mitoxantrone hydrochloride liposome injection combined with capecitabine versus capecitabine monotherapy in subjects with recurrent and/or metastatic nasopharyngeal carcinoma.

(II) Study method

1. Study design

This study consists of a screening period (-28 days to 0 days), a treatment period, an end-of-treatment (EOT) visit period, and a follow-up period.

Subjects with recurrent and/or metastatic nasopharyngeal carcinoma who have failed first-line or second-line platinum-based therapy will be enrolled. After screening, eligible subjects are stratified-randomized (stratification factors: (1) ECOG score: 0 vs. 1; (2) whether a liver metastasis is present: any metastasis is present vs. all metastases are not present; (3) gender: male vs. female) in a 1:1 ratio into an experimental group and a control group.

The experimental group receives treatment with mitoxantrone hydrochloride liposome injection (20 mg/m$^2$) combined with capecitabine (1000 mg/m$^2$, twice daily), with every 3 weeks as a treatment cycle. The mitoxantrone hydrochloride liposome is administered on day 1 of each cycle, and capecitabine is administered on days 1 to 14. After 8 cycles of treatment with the mitoxantrone hydrochloride liposome, the investigator will assess the patient's risk-benefit ratio to determine whether to continue treatment until disease progression or intolerance, and capecitabine monotherapy as maintenance treatment. The control group receives capecitabine monotherapy, with every 3 weeks as a treatment cycle. Capecitabine monotherapy is administered on days 1 to 14 of each cycle. Sparse blood collection is conducted for all subjects in the experimental group.

The minimum allowable dose reduction principle is detailed in Table 1. If more dose reduction is required, the treatment with the study drug must be discontinued. Once the dose is reduced, the dose level should be maintained for all subsequent cycles, or further reduced if necessary. Dose escalation is not allowed in this study. If a subject experiences the following drug-related adverse events after administration, the dose of mitoxantrone may be reduced in the next treatment cycle based on the investigator's assessment. The dose level of the mitoxantrone hydrochloride liposome can be reduced only once per cycle. If necessary, the dose of capecitabine may be reduced within this treatment cycle.

Table 1. Dosage modification regimen

|  | Mitoxantrone liposome | Capecitabine |
|---|---|---|
| Starting dose | 20 mg/m$^2$ | 1000 mg/m$^2$ |
| The first reduction | 16 mg/m$^2$ | 75%/750 mg/m$^2$ |
| The second reduction | 12 mg/m$^2$ | 50%/500 mg/m$^2$ |

During the study, the Independent Data Monitoring Committee (IDMC) will conduct safety assessments after two cycles of treatment for the first 40 subjects enrolled(about 20 per group) and throughout the study. Enrollment will continue during the safety assessment period. All subjects will be treated until disease progression according to the RECIST1.1 criteria, intolerable toxicity, withdrawal of informed consent, initiation of new anti-tumor therapy, loss to follow-up, death, or end of study, whichever occurs first. If the subject has no more treatment options after the investigator first determines that the subject has experienced disease progression, and the investigator determines that the subject can continue to benefit from the trial treatment, the subject may continue to receive the trial treatment in this trial until disease progression as assessed by the IRC or the investigator determines that the subject no longer benefits.

2. Trial population:

2.1. Inclusion criteria:

Subjects must meet all of the following criteria to be eligible for enrollment into this study:

1) The subject voluntarily participated in the study and signed informed consent.
2) Age $\geq$ 18 years.
3) Nasopharyngeal carcinoma confirmed by histopathology .
4) Recurrent and/or metastatic nasopharyngeal carcinoma that failed prior first-line or second-line platinum-based therapy.
5) At least one evaluable lesion at baseline according to the RECIST 1.1 criteria.
6) ECOG score of 0-1.
7) Toxicity of prior anti-tumor therapy has not recovered to $\leq$ grade 1 (except for alopecia, pigmentation, or other toxicities deemed by the investigator to pose no safety risk to the subject).
8) The subject's laboratory test values meet the following requirements (The following indicators can be achieved without blood transfusion or growth factor therapy within 14 days before the screening sample collection):

- $$\text{Absolute neutrophil count (ANC)} \geq 1.5 \times 10^9/\text{L};$$

- 
- $$\text{Hemoglobin (Hb)} \geq 9.0 \text{ g/dL};$$

$$\text{Platelet count} \geq 90 \times 10^9/\text{L};$$

- $$\text{Creatinine} \leq 1.5 \times \text{ULN};$$

- Total bilirubin $\leq$ 1.5 $\times$ ULN ($\leq$ 3 $\times$ ULN for subjects with liver metastases);
- Alanine aminotransferase (ALT)/aspartate aminotransferase (AST) $\leq$ 2.5 $\times$ ULN ($\leq$ 5 $\times$ ULN for subjects with liver metastases);
- Coagulation: prothrombin time (PT), International normalized ratio (INR) $\leq$ 1.5 $\times$ ULN.

9) Female subjects must have a negative blood HCG test (except for menopause and hysterectomy). Female subjects of childbearing potential and their partners must use effective contraceptive measures (e.g., combination hormones [containing estrogen and progesterone] to suppress ovulation, progestogen contraception to suppress ovulation, intrauterine device, intrauterine hormone release system, bilateral tubal ligation, vasectomy, avoidance of sexual activity, etc) during the trial and within 6 months after the last dose.

10) Male subjects and their partners agree to use one of the contraceptive measures described in criterion 9.

2.2. Exclusion criteria:

Subjects who meet any of the following criteria will not be eligible for participation in the study:

1) Severe allergy to mitoxantrone or liposomes; history of severe or unexpected reactions to fluorouracils or known allergy to fluorouracils.

2) Active leptomeningeal disease or poorly controlled brain metastase. Subjects with suspected or confirmed brain metastases are allowed to be enrolled if they do not have significant symptoms, the results of imaging conducted $\geq 28$ days prior to the first dose of the study drug show stable disease, and no treatment (e.g., radiotherapy, surgery, or corticosteroid treatment) is required to control symptoms of brain metastases.

3) Expected survival < 3 months.

4) Active hepatitis B (HbsAg or HBcAb positive and HBV DNA $\geq 2000$ IU/mL), active hepatitis C (HCV antibody positive and HCV RNA is above the lower limit of detection), and HIV antibody positive.

5) Active bacterial infections, fungal infections, viral infections, or interstitial pneumonia requiring systemic treatment within 1 week prior to the initial administration of the study drug.

6) Received anti-tumor therapies such as chemotherapy, small molecule inhibitors, immunotherapy (e.g., interleukins, interferons, or thymosin) within 4 weeks or 5 half-lives (whichever is shorter, but at least 2 weeks) prior to initial administration; Received Chinese patent drugs with anti-tumor activity within 14 days prior to initial administration.

7) Received another investigational drugs within 4 weeks prior to initial administration.

8) Those who received major surgery within 3 months prior to initial administration or plan to receive major surgery during the study.

9) Severe embolic events, such as cerebrovascular accidents (including transient ischemic attacks) and pulmonary embolism, within the past 6 months.

10) Active malignant tumors within 2 years prior to initial administration, except for the nasopharyngeal carcinoma studied in this trial and any locally curable tumor that has been radically treated (e.g., resected basal cell or squamous cell skin cancer, superficial bladder cancer, or cervical or breast carcinoma in situ).

11) Heart function abnormalities, including:

- Long QTc syndrome or QTc interval > 480 ms;
- Complete left bundle branch block, degree II or degree III atrioventricular block;
- Severe, uncontrolled arrhythmia requiring medication;
- History of chronic congestive heart failure with NYHA class $\geq 3$;
- Cardiac ejection fraction less than 50% or less than the lower limit of the laboratory test value within the past 6 months;
- Grade $\geq 3$ heart valvular heart disease as defined by CTCAE version 5.0;
- Uncontrolled hypertension (defined as systolic pressure measurement > 160 mmHg or diastolic pressure measurement > 90 mmHg under drug control);
- History of myocardial infarction, unstable angina, severe pericardial disease, and electrocardiographic evidence of acute ischemic or active conduction system abnormalities within 6 months prior to screening.

12) Prior treatment with doxorubicin or other anthracyclines, with the cumulative doxorubicin dose exceeding 350 mg/m$^2$ (anthracycline equivalent dose calculation: 1 mg doxorubicin = 2 mg epirubicin = 2 mg daunorubicin = 0.5 mg idarubicin = 0.45 mg mitoxantrone).

13) Women who are pregnant or breastfeeding.

14) Any severe and/or uncontrollable disease, and other diseases that, in the investigator's opinion, may affect the subject's participation in this study (including but not limited to poorly controlled diabetes, kidney disease requiring dialysis, severe liver disease, life-threatening autoimmune diseases and hemorrhagic diseases, drug abuse, neurological diseases, etc.).

15) Other situations that the investigator determines to be inappropriate for participation.

3. Evaluation criteria

Primary study endpoints:

- Progression-free survival (PFS) as assessed by the Independent Review Committee (IRC)
- Overall survival (OS)

Secondary study endpoints:

- Objective response rate (ORR), disease control rate (DCR), and duration of response (DOR) as assessed by the investigator and the IRC
- Progression-free survival (PFS) as assessed by the investigator
- Frequency and severity of AEs (NCI CTCAE 5.0); changes in vital signs, physical examinations, electrocardiograms, and laboratory findings
- Total mitoxantrone and free mitoxantrone plasma concentrations.

Note: The abbreviations described above have the following meanings:
PFS: progression-free survival, defined as the time between the initial treatment of study drug and the date of disease progression (PD) or death, whichever occurs first.
OS: overall survival, defined as the time from the initial treatment of the study drug to death from any cause.
ORR: objective response rate, defined as the proportion of subjects achieving complete response (CR) and partial response (PR).
DCR: disease control rate, defined as the proportion of subjects achieving complete response (CR), partial response (PR), and stable disease (SD).
DOR: duration of response, defined as the time from the first assessment of CR or PR to the first assessment of PD or death from any cause.

(III) Study results

Up to July 31, 2023, total of 62 subjects enrolled, including 32 subjects in the experimental group and 30 subjects in the control group; 4 subjects were end of the study (in the control group, one subject withdrew consent, and two subjects died; in the experimental group, one subject died), and 58 subjects remained in the study.

1. Baseline characteristics of subjects

Of the 32 subjects in the experimental group, 56.9% were patients with metastatic nasopharyngeal carcinoma, 18.8% were patients with recurrent nasopharyngeal carcinoma, and 34.4% were patients with recurrent and metastatic nasopharyngeal carcinoma; of the 30 subjects in the control group, 30% were patients with metastatic nasopharyngeal carcinoma, 13.3% were patients with recurrent nasopharyngeal carcinoma, and 56.7% were patients with recurrent and metastatic nasopharyngeal carcinoma (see Table 2 for details).

Table 2. Baseline characteristics of subjects

| Baseline characteristics | Experimental group | | Control group | |
|---|---|---|---|---|
| | N | Percentage | N | Percentage |
| | 32 | / | 30 | / |
| Sex | Male | 29 | 90.6% | 22 | 73.3% |
| | Female | 3 | 9.4% | 8 | 26.7% |
| ECOG | 0 | 10 | 31.3% | 8 | 26.7% |
| | 1 | 22 | 68.8% | 22 | 73.3% |
| PD-1 treatment | Provided | 19 | 59.4% | 21 | 70% |
| | None | 13 | 40.6% | 9 | 30% |

(continued)

| Baseline characteristics | | Experimental group | | Control group | |
|---|---|---|---|---|---|
| | | N | Percentage | N | Percentage |
| | | 32 | / | 30 | / |
| Liver metastasis | Yes | 14 | 43.8% | 13 | 43.3% |
| | No | 18 | 56.3% | 17 | 56.7% |
| Prior lines of therapy | 1 line | 16 | 50% | 22 | 73.3% |
| | 2 lines | 16 | 50% | 8 | 26.7% |
| Pathological type | Keratinizing squamous cell carcinoma | 4 | 12.5% | 3 | 10% |
| | Non-keratinizing squamous cell carcinoma | 27 | 84.4% | 27 | 90% |
| | Basal-like squamous cell carcinoma | 0 | 0 | 0 | 0 |
| | Others | 1 | 3.1% | 0 | 0 |
| Site of tumor metastasis | Liver | 15 | 56.9% | 13 | 43.3% |
| | Lung | 6 | 18.8% | 11 | 36.7% |
| | Bone | 15 | 56.9% | 13 | 43.3% |
| | Others | 4 | 12.5% | 11 | 36.7% |
| Tumor status | Distant metastasis only | 15 | 56.9% | 9 | 30% |
| | Local recurrence only | 6 | 18.8% | 4 | 13.3% |
| | Local recurrence with distant metastasis | 11 | 34.4% | 17 | 56.7% |
| Tumor stage | IVa | 5 | 15.6% | 6 | 20% |
| | IVb | 26 | 81.3% | 23 | 76.7% |
| | Others | 1 | 3.1% | 1 | 3.3% |
| EBV DNA positive | Yes | 21 | 65.6% | 25 | 83.3% |
| | No | 11 | 34.4% | 5 | 16.7% |

2. Efficacy evaluation

By July 31, 2023, a total of 9 subjects in the experimental group were evaluated for best overall response (BOR). Of them, one achieved PD (11.1%), three achieved SD (33.3%), and five achieved PR (55.6%).

Table 3. Best-overall-response evaluations of the experimental group

| Screening number | Sex | ECOG | Number of lines received | Prior treatments | Metastasis site(s) | Best overall response |
|---|---|---|---|---|---|---|
| S29001 | Male | 0 | 2 | First line: anlotinib + sintilimab + albumin-bound paclitaxel + cisplatin*2<br>Second line: TAB-BX102 (CD73)*4 | Liver and bone | PR |
| S01002 | Male | 1 | 1 | First line: albumin-bound paclitaxel + lobaplatin + camrelizumab*5 → lobaplatin + camrelizumab (concurrent)*2 → camrelizumab (maintenance)*16 | Liver and lung | PR |

(continued)

| Screening number | Sex | ECOG | Number of lines received | Prior treatments | Metastasis site(s) | Best overall response |
|---|---|---|---|---|---|---|
| S01003 | Male | 1 | 2 | First line: anlotinib/placebo + GP*6 → anlotinib/placebo maintenance*20 Second line: albumin-bound paclitaxel + carboplatin + tislelizumab*5 → tislelizumab maintenance*4 | Liver | SD |
| S08001 | Male | 1 | 2 | First line: gemcitabine + nedaplatin + tislelizumab*6 → tislelizumab + gemcitabine*2 → tislelizumab* 1; Second line: JS004 mAb*13 | Liver and mediastinal lymph node | PR |
| S01004 | Female | 1 | 2 | First line: docetaxel + cisplatin + fluorouracil*4 → cisplatin (concur-rent)*2 Second line: carboplatin + paclitaxel + toripalimab* 3 | Liver and abdominal lymph node | PR |
| S28001 | Female | 1 | 1 | First line: docetaxel + cisplatin + fluorouracil*3 + concurrent radical radiotherapy with nimotuzumab combined with cisplatin | Bone and lung | SD |
| S28002 | Male | 1 | 1 | First line: gemcitabine + cisplatin + toripalimab*6 + 1 week of toripalimab maintenance | Cervical lymph node | PR |
| S13002 | Male | 1 | 1 | First line: gemcitabine + cisplatin*6 → AK105 maintenance therapy 7 | Lung | PD |
| S23001 | Male | 0 | 2 | First line: gemcitabine + cisplatin induction chemotherapy → nedaplatin concurrent chemotherapy → nimotu-zumab maintenance therapy; Second line: toripalimab + tegafur, gimeracil, and oteracil potassium*2 | Liver | SD |

A total of 5 subjects in the control group were evaluated for best overall response (BOR). Of them, three achieved PD (60%), one achieved SD (20%), and one achieved PR (20%).

Table 4. Best-overall-response evaluations of the control group

| Screening number | Sex | ECOG | Number of lines received | Prior treatments | Metastasis site(s) | Best overall response |
|---|---|---|---|---|---|---|
| S01005 | Female | 0 | 1 | Induction chemotherapy: nedaplatin + fluorouracil*3 First line: gemcitabine + cisplatin*3 | Liver and lung | SD |
| S32001 | Female | 1 | 1 | First line: TP*2 + TP concurrent radiotherapy*1 + TP maintenance chemotherapy*1 | Liver | PD |
| S13001 | Male | 1 | 1 | First line: toripalimab + cisplatin + gemcitabine* 6 | Liver, spleen, and bone | PD |

(continued)

| Screening number | Sex | ECOG | Number of lines received | Prior treatments | Metastasis site(s) | Best overall response |
|---|---|---|---|---|---|---|
| S24001 | Male | 1 | 2 | First line: docetaxel + nedaplatin*3 → concurrent nedaplatin*2 → concurrent nimotuzumab* 5; Second line: toripalimab + gemcitabine + nedaplatin*2; | Bone | PR |
| S14001 | Male | 1 | 2 | First line: docetaxel + nedaplatin + Endostar*3 induction chemotherapy, 3 cycles of nedaplatin concurrent chemotherapy, and S-1(tegafur, gimeracil, and oteracil potassium) maintenance therapy; Second line: gemcitabine + cisplatin + toripalimab + Endostar*6 | Liver and bone | PD |

From the above results, the mitoxantrone liposome combined with capecitabine showed better efficacy than capecitabine monotherapy in treating recurrent and/or metastatic nasopharyngeal carcinoma.

3. Safety evaluation

In the experimental group, a total of 9 subjects experienced serious adverse events (SAEs), including hematologic adverse events, such as white blood cell count decreased, neutrophil count decreased, platelet count decreased, and anemia, as well as hyponatremia, hypokalemia, pulmonary infection, etc., and no severe cardiac adverse effects were observed. In the control group, a total of 3 subjects experienced SAEs, including bacterial pneumonia, severe pneumonia, post-cardiac arrest resuscitation syndrome, etc.

Table 5. An overview of SAEs in the control and experimental groups

| SAE | Experimental group (N = 32) n (%) | Control group (N = 30) n (%) |
|---|---|---|
| Posterior-circulation ischemia | 0 | 1 (3.3%) |
| Death from unknown cause | 0 | 1 (3.3%) |
| White blood cell count decreased | 1 (3.1%) | 0 |
| Neutrophil count decreased | 1 (3.1%) | 0 |
| Platelet count decreased | 1 (3.1%) | 0 |
| Hyponatremia | 2 (6.3%) | 0 |
| Upper airway | 1 (3.1%) | 0 |
| obstruction | | |
| General fatigue | 1 (3.1%) | 0 |
| Dysphagia | 0 | 1 (3.3%) |
| Bacterial pneumonia | 0 | 1 (3.3%) |
| Severe pneumonia | 0 | 1 (3.3%) |
| Post-cardiac arrest resuscitation syndrome | 0 | 1 (3.3%) |
| Sinusitis | 1 (3.1%) | 0 |
| Cancer pain | 1 (3.1%) | 0 |
| Acute epiglottitis | 1 (3.1%) | 0 |
| Hypokalemia | 1 (3.1%) | 0 |

(continued)

| SAE | Experimental group (N = 32) n (%) | Control group (N = 30) n (%) |
|---|---|---|
| Anemia | 1 (3.1%) | 0 |
| Oralmucositis | 0 | 1 (3.3%) |
| Pulmonary infection | 1 (3.1%) | 0 |

**Claims**

1. Use of a mitoxantrone liposome and capecitabine for manufacturing a medicament for the treatment of nasopharyngeal carcinoma.

2. Use of a mitoxantrone liposome for manufacturing a medicament for improving efficacy of capecitabine in treating nasopharyngeal carcinoma.

3. The use according to claim 1 or 2, wherein the nasopharyngeal carcinoma is recurrent and/or metastatic nasopharyngeal carcinoma; preferably, the recurrent and/or metastatic nasopharyngeal carcinoma is recurrent and/or metastatic nasopharyngeal carcinoma that has previously undergone first-line or second-line platinum-based therapy.

4. The use according to any one of claims 1-3, wherein the mitoxantrone liposome has one or more of the following properties:

    (i) the mitoxantrone liposome is a mitoxantrone hydrochloride liposome;
    (ii) the mitoxantrone liposome has a particle size of about 30-80 nm, e.g., about 35-75 nm, about 40-70 nm, about 40-60 nm, or about 60 nm;
    (iii) mitoxantrone forms a poorly soluble precipitate with multivalent counterions (e.g., sulfate anion , citrate anion, or phosphate anion) in the liposome; and
    (iv) a phospholipid bilayer in the mitoxantrone liposome comprises a phospholipid with a phase transition temperature (Tm) higher than body temperature, wherein the phospholipid is selected from hydrogenated soybean lecithin, phosphatidyl choline, hydrogenated yolk lecithin, dipalmitoyl lecithin, distearoyl lecithin, and any combination thereof; preferably, the phospholipid is selected from hydrogenated soybean lecithin, cholesterol, and polyethylene glycol 2000-modified distearoyl phosphatidyl ethanolamine; more preferably, the phospholipid is hydrogenated soybean lecithin, cholesterol, and polyethylene glycol 2000-modified distearoyl phosphatidyl ethanolamine in a mass ratio of about 3:1:1.

5. The use according to any one of claims 1-4, wherein the mitoxantrone liposome is a mitoxantrone hydrochloride liposome.

6. The use according to any one of claims 1-5, wherein the mitoxantrone liposome is a liquid injection comprising an active ingredient at 0.5-5 mg/mL, preferably 1-2 mg/mL, and more preferably 1 mg/mL, on the basis of mitoxantrone.

7. The use according to any one of claims 1-6, wherein capecitabine is a tablet.

8. The use according to any one of claims 1-7, wherein the medicament further comprises other medicaments for the treatment of nasopharyngeal carcinoma.

9. A medicament for the treatment of nasopharyngeal carcinoma, comprising a mitoxantrone liposome and capecitabine.

10. A medicament for improving efficacy of capecitabine in treating nasopharyngeal carcinoma, comprising a mitoxantrone liposome.

11. The medicament according to claim 9 or 10, wherein the nasopharyngeal carcinoma is recurrent and/or metastatic nasopharyngeal carcinoma; preferably, the recurrent and/or metastatic nasopharyngeal carcinoma is recurrent and/or metastatic nasopharyngeal carcinoma that has previously undergone first-line or second-line platinum-based therapy.

12. The medicament according to any one of claims 9-11, wherein the mitoxantrone liposome has one or more of the following properties:

    (i) the mitoxantrone liposome is a mitoxantrone hydrochloride liposome;
    (ii) the mitoxantrone liposome has a particle size of about 30-80 nm, e.g., about 35-75 nm, about 40-70 nm, about 40-60 nm, or about 60 nm;
    (iii) mitoxantrone forms a poorly soluble precipitate with multivalent counterions (e.g., sulfate anion, citrate anion, or phosphate anion) in the liposome ; and
    (iv) a phospholipid bilayer in the mitoxantrone liposome comprises a phospholipid with a phase transition temperature (Tm) higher than body temperature, wherein the phospholipid is selected from hydrogenated soybean lecithin, phosphatidyl choline, hydrogenated yolk lecithin, dipalmitoyl lecithin, distearoyl lecithin, and any combination thereof; preferably, the phospholipid is selected from hydrogenated soybean lecithin, cholesterol, and polyethylene glycol 2000-modified distearoyl phosphatidyl ethanolamine; more preferably, the phospholipid is hydrogenated soybean lecithin, cholesterol, and polyethylene glycol 2000-modified distearoyl phosphatidyl ethanolamine in a mass ratio of about 3:1:1.

13. The medicament according to any one of claims 9-12, wherein the mitoxantrone liposome is a mitoxantrone hydrochloride liposome.

14. The medicament according to any one of claims 9-13, wherein the mitoxantrone liposome is a liquid injection comprising an active ingredient at 0.5-5 mg/mL, preferably 1-2 mg/mL, and more preferably 1 mg/mL, on the basis of mitoxantrone.

15. The medicament according to any one of claims 9-14, wherein capecitabine is a tablet.

16. The medicament according to any one of claims 9-15, wherein the mitoxantrone liposome is administered by injection; preferably, the mitoxantrone liposome is administered by injection at a therapeutically effective dose of 8-30 mg/m$^2$, preferably 12-24 mg/m$^2$, and more preferably 12-20 mg/m$^2$ on day 1 of each administration cycle.

17. The medicament according to any one of claims 9-16, wherein capecitabine is administered orally; preferably, capecitabine is administered orally at a dose of 500 mg/m$^2$, 750 mg/m$^2$, or 1000 mg/m$^2$ twice daily on days 1-14 of each administration cycle.

18. The medicament according to any one of claims 9-17, wherein the treatment comprises administering the mitoxantrone liposome by injection at a therapeutically effective dose of 8-30 mg/m$^2$ and administering capecitabine orally at a dose of 500 mg/m$^2$, 750 mg/m$^2$, or 1000 mg/m$^2$ on day 1 of each administration cycle, and then continuing administering capecitabine orally at a dose of 500 mg/m$^2$, 750 mg/m$^2$, or 1000 mg/m$^2$ on days 2-14 of the cycle.

19. The medicament according to any one of claims 9-18, wherein the treatment comprises administering the mitoxantrone liposome by injection at a therapeutically effective dose of 20 mg/m$^2$ once and administering capecitabine orally at a dose of 1000 mg/m$^2$ twice on day 1 of each administration cycle, and then continuing administering capecitabine orally at a dose of 1000 mg/m$^2$ twice daily on days 2-14 of the cycle.

20. The medicament according to any one of claims 16-19, wherein the administration cycle is three weeks.

21. The medicament according to any one of claims 9-20, further comprising other medicaments for the treatment of nasopharyngeal carcinoma.

22. A method of the treatment of nasopharyngeal carcinoma, comprising administering to a patient with nasopharyngeal carcinoma a therapeutically effective dose of a mitoxantrone liposome and capecitabine.

23. A method for improving efficacy of capecitabine in treating nasopharyngeal carcinoma, comprising administering capecitabine to a patient with nasopharyngeal carcinoma in combination with a therapeutically effective dose of a mitoxantrone liposome.

24. The method according to claim 22 or 23, wherein the nasopharyngeal carcinoma is recurrent and/or metastatic nasopharyngeal carcinoma; preferably, the recurrent and/or metastatic nasopharyngeal carcinoma is recurrent and/or metastatic nasopharyngeal carcinoma that has previously undergone first-line or second-line platinum-based therapy.

25. The method according to any one of claims 22-24, wherein the mitoxantrone liposome has one or more of properties:

(i) the mitoxantrone liposome is a mitoxantrone hydrochloride liposome;
(ii) the mitoxantrone liposome has a particle size of about 30-80 nm, e.g., about 35-75 nm, about 40-70 nm, about 40-60 nm, or about 60 nm;
(iii) mitoxantrone forms a poorly soluble precipitate with multivalent counterions (e.g., sulfate anion, citrate anion, or phosphate anion) in the liposome; and
(iv) a phospholipid bilayer in the mitoxantrone liposome comprises a phospholipid with a phase transition temperature (Tm) higher than body temperature, wherein the phospholipid is selected from hydrogenated soybean lecithin, phosphatidyl choline, hydrogenated yolk lecithin, dipalmitoyl lecithin, distearoyl lecithin, and any combination thereof; preferably, the phospholipid is selected from hydrogenated soybean lecithin, cholesterol, and polyethylene glycol 2000-modified distearoyl phosphatidyl ethanolamine; more preferably, the phospholipid is hydrogenated soybean lecithin, cholesterol, and polyethylene glycol 2000-modified distearoyl phosphatidyl ethanolamine in a mass ratio of about 3:1:1.

26. The method according to any one of claims 22-25, wherein the mitoxantrone liposome is a mitoxantrone hydrochloride liposome.

27. The method according to any one of claims 22-26, wherein the mitoxantrone liposome is a liquid injection comprising an active ingredient at 0.5-5 mg/mL, preferably 1-2 mg/mL, and more preferably 1 mg/mL, on the basis of mitoxantrone.

28. The method according to any one of claims 22-27, wherein capecitabine is a tablet.

29. The method according to any one of claims 22-28, wherein the mitoxantrone liposome is administered by injection; preferably, the mitoxantrone liposome is administered by injection at a therapeutically effective dose of 8-30 mg/m$^2$, preferably 12-24 mg/m$^2$, and more preferably 12-20 mg/m$^2$ on day 1 of each administration cycle.

30. The method according to any one of claims 22-29, wherein capecitabine is administered orally; preferably, capecitabine is administered orally at a dose of 500 mg/m$^2$, 750 mg/m$^2$, or 1000 mg/m$^2$ twice daily on days 1-14 of each administration cycle.

31. The method according to any one of claims 22-30, comprising administering the mitoxantrone liposome by injection at a therapeutically effective dose of 8-30 mg/m$^2$ and administering capecitabine orally at a dose of 500 mg/m$^2$, 750 mg/m$^2$, or 1000 mg/m$^2$ on day 1 of each administration cycle, and then continuing administering capecitabine orally at a dose of 500 mg/m$^2$, 750 mg/m$^2$, or 1000 mg/m$^2$ on days 2-14 of the cycle.

32. The method according to any one of claims 22-31, comprising administering the mitoxantrone liposome by injection at a therapeutically effective dose of 20 mg/m$^2$ once and administering capecitabine orally at a dose of 1000 mg/m$^2$ twice on day 1 of each administration cycle, and then continuing administering capecitabine orally at a dose of 1000 mg/m$^2$ twice daily on days 2-14 of the cycle.

33. The method according to any one of claims 29-32, wherein the administration cycle is three weeks.

34. A pharmaceutical combination for the treatment of nasopharyngeal carcinoma, comprising a mitoxantrone liposome and capecitabine.

35. The pharmaceutical combination according to claim 34, wherein the nasopharyngeal carcinoma is recurrent and/or metastatic nasopharyngeal carcinoma; preferably, the recurrent and/or metastatic nasopharyngeal carcinoma is recurrent and/or metastatic nasopharyngeal carcinoma that has previously undergone first-line or second-line platinum-based therapy.

36. The pharmaceutical combination according to claim 34 or 35, wherein the mitoxantrone liposome has one or more of the following properties:

(i) the mitoxantrone liposome is a mitoxantrone hydrochloride liposome;

(ii) the mitoxantrone liposome has a particle size of about 30-80 nm, e.g., about 35-75 nm, about 40-70 nm, about 40-60 nm, or about 60 nm;

(iii) mitoxantrone forms a poorly soluble precipitate with multivalent counterions (e.g., sulfate anion, citrate anion, or phosphate anion) in the liposome; and

(iv) a phospholipid bilayer in the mitoxantrone liposome comprises a phospholipid with a phase transition temperature (Tm) higher than body temperature, wherein the phospholipid is selected from hydrogenated soybean lecithin, phosphatidyl choline, hydrogenated yolk lecithin, dipalmitoyl lecithin, distearoyl lecithin, and any combination thereof; preferably, the phospholipid is selected from hydrogenated soybean lecithin, cholesterol, and polyethylene glycol 2000-modified distearoyl phosphatidyl ethanolamine; more preferably, the phospholipid is hydrogenated soybean lecithin, cholesterol, and polyethylene glycol 2000-modified distearoyl phosphatidyl ethanolamine in a mass ratio of about 3:1:1.

37. The pharmaceutical combination according to any one of claims 34-36, wherein the mitoxantrone liposome is a mitoxantrone hydrochloride liposome.

38. The pharmaceutical combination according to any one of claims 34-37, wherein the mitoxantrone liposome is a liquid injection comprising an active ingredient at 0.5-5 mg/mL, preferably 1-2 mg/mL, and more preferably 1 mg/mL, on the basis of mitoxantrone.

39. The pharmaceutical combination according to any one of claims 34-38, wherein capecitabine is a tablet.

40. The pharmaceutical combination according to any one of claims 34-39, wherein the mitoxantrone liposome is administered by injection; preferably, the mitoxantrone liposome is administered by injection at a therapeutically effective dose of 8-30 mg/m$^2$, preferably 12-24 mg/m$^2$, and more preferably 12-20 mg/m$^2$ on day 1 of each administration cycle.

41. The pharmaceutical combination according to any one of claims 34-40, wherein capecitabine is administered orally; preferably, capecitabine is administered orally at a dose of 500 mg/m$^2$, 750 mg/m$^2$, or 1000 mg/m$^2$ twice daily on days 1-14 of each administration cycle.

42. The pharmaceutical combination according to any one of claims 34-41, wherein the treatment comprises administering the mitoxantrone liposome by injection at a therapeutically effective dose of 8-30 mg/m$^2$ and administering capecitabine orally at a dose of 500 mg/m$^2$, 750 mg/m$^2$, or 1000 mg/m$^2$ on day 1 of each administration cycle, and then continuing administering capecitabine orally at a dose of 500 mg/m$^2$, 750 mg/m$^2$, or 1000 mg/m$^2$ on days 2-14 of the cycle.

43. The pharmaceutical combination according to any one of claims 34-42, wherein the treatment comprises administering the mitoxantrone liposome by injection at a therapeutically effective dose of 20 mg/m$^2$ once and administering capecitabine orally at a dose of 1000 mg/m$^2$ twice on day 1 of each administration cycle, and then continuing administering capecitabine orally at a dose of 1000 mg/m$^2$ twice daily on days 2-14 of the cycle.

44. The pharmaceutical combination according to any one of claims 40-43, wherein the administration cycle is three weeks.

45. A kit, comprising a mitoxantrone liposome and capecitabine.

46. The kit according to claim 45, wherein the mitoxantrone liposome has one or more of the following properties:

(i) the mitoxantrone liposome is a mitoxantrone hydrochloride liposome;

(ii) the mitoxantrone liposome has a particle size of about 30-80 nm, e.g., about 35-75 nm, about 40-70 nm, about 40-60 nm, or about 60 nm;

(iii) mitoxantrone forms a poorly soluble precipitate with multivalent counterions (e.g., sulfate anion, citrate anion, or phosphate anion) in the liposome; and

(iv) a phospholipid bilayer in the mitoxantrone liposome comprises a phospholipid with a phase transition temperature (Tm) higher than body temperature, wherein the phospholipid is selected from hydrogenated soybean lecithin, phosphatidyl choline, hydrogenated yolk lecithin, dipalmitoyl lecithin, distearoyl lecithin, and any combination thereof; preferably, the phospholipid is selected from hydrogenated soybean lecithin, cholesterol, and polyethylene glycol 2000-modified distearoyl phosphatidyl ethanolamine; more preferably, the phospholipid is hydrogenated soybean lecithin, cholesterol, and polyethylene glycol 2000-modified distearoyl phosphatidyl ethanolamine in a mass ratio of about 3:1:1.

47. The kit according to claim 45 or 46, wherein the mitoxantrone liposome is a mitoxantrone hydrochloride liposome.

48. The kit according to any one of claims 45-47, wherein the mitoxantrone liposome is a liquid injection comprising an active ingredient at 0.5-5 mg/mL, preferably 1-2 mg/mL, and more preferably 1 mg/mL, on the basis of mitoxantrone.

49. The kit according to any one of claims 45-48, wherein capecitabine is a tablet.

# EP 4 582 088 A1

## INTERNATIONAL SEARCH REPORT

| | International application No. |
|---|---|
| | **PCT/CN2023/115149** |

| A. | CLASSIFICATION OF SUBJECT MATTER |
|---|---|

A61K 31/7068(2006.01)i; A61P35/00(2006.01)i; A61P35/04(2006.01)i; A61K31/136(2006.01)i

According to International Patent Classification (IPC) or to both national classification and IPC

| B. | FIELDS SEARCHED |
|---|---|

Minimum documentation searched (classification system followed by classification symbols)

IPC:A61K A61P

Documentation searched other than minimum documentation to the extent that such documents are included in the fields searched

Electronic data base consulted during the international search (name of data base and, where practicable, search terms used)

CNABS; NCBI; JPABS; TWABS; CNTXT; WOTXT; USTXT; EPTXT; CJFD; CNKI; VEN; DWPI; SIPOABS; PUBMED; ELSEVIER; Patentics; STN: 鼻咽癌, 米托蒽醌, 卡培他滨, 多恩达, mitoxantrone, novantron, capecitabine, xelox, Nasopharyngeal Cancer

| C. | DOCUMENTS CONSIDERED TO BE RELEVANT |
|---|---|

| Category* | Citation of document, with indication, where appropriate, of the relevant passages | Relevant to claim No. |
|---|---|---|
| X | WO 2022127760 A1 (CSPC ZHONGQI PHARMACEUTICAL TECH SHIJIAZHUANG CO., LTD.) 23 June 2022 (2022-06-23) <br> description, paragraphs [0015]-[0024] and [0350] | 10-21 |
| A | WO 2022127760 A1 (CSPC ZHONGQI PHARMACEUTICAL TECH SHIJIAZHUANG CO., LTD.) 23 June 2022 (2022-06-23) <br> description, paragraphs [0015]-[0024] and [0350] | 1-9, 34-49 |
| A | WO 2004056379 A1 (PANAGIN PHARMACEUTICALS INC. et al.) 08 July 2004 (2004-07-08) <br> claims 1-12 | 1-21, 34-49 |
| PX | 小久 (Xiaojiu). "[鼻咽癌] 上市药品多恩达注射液联合卡培他滨治疗鼻咽癌患者招募 (Non-official translation: [Nasopharyngeal carcinoma] Recruitment of Patients with Nasopharyngeal Carcinoma Treated with Listed Drug Duoenda Injection Combined with Capecitabine)" <br> *https://www.yuge.com/news/getNewsById/35209463.htm*, 07 July 2023 (2023-07-07), pp. 1-2 <br> paragraphs 1-2 | 1-21, 34-49 |

| ☐ Further documents are listed in the continuation of Box C. | ☑ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **01 December 2023** | **05 December 2023** |

| Name and mailing address of the ISA/CN | Authorized officer |
|---|---|
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/115149** |

**Box No. II      Observations where certain claims were found unsearchable (Continuation of item 2 of first sheet)**

This international search report has not been established in respect of certain claims under Article 17(2)(a) for the following reasons:

1. ☑  Claims Nos.: **22-33**
   because they relate to subject matter not required to be searched by this Authority, namely:

   The subject matter of claim 22 is: a method for treating nasopharyngeal carcinoma; the subject matter of claim 23 is: a method for improving the curative effect of capecitabine for treating nasopharyngeal carcinoma; and dependent claims 24-33 refer to the preceding subject matter. The above-mentioned subject matter relates to a treatment method, which falls within subject matter for which no search is required by the International Searching Authority as defined in PCT Rule 39.1(iv).

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

**INTERNATIONAL SEARCH REPORT**
Information on patent family members

International application No.

**PCT/CN2023/115149**

| Patent document cited in search report | | | Publication date (day/month/year) | Patent family member(s) | | | Publication date (day/month/year) |
|---|---|---|---|---|---|---|---|
| WO | 2022127760 | A1 | 23 June 2022 | AU | 2021399438 | A1 | 06 July 2023 |
| | | | | CA | 3202019 | A1 | 23 June 2022 |
| | | | | KR | 20230107637 | A | 17 July 2023 |
| | | | | EP | 4265243 | A1 | 25 October 2023 |
| WO | 2004056379 | A1 | 08 July 2004 | AU | 2003291896 | A1 | 14 July 2004 |

Form PCT/ISA/210 (patent family annex) (July 2022)

EP 4 582 088 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008080367 A1

- CN 200610102339

### Non-patent literature cited in the description

- **MA SX** ; **ZHOU T** ; **HUANG Y et al.** The efficacy of first line chemotherapy in recurrent or metastatic nasopharyngeal carcinoma: a systematic review and meta analysis.. *Ann Transl Med*, 2018, vol. 6 (11), 201